# EUROPÄISCHE PATENTANMELDUNG

(11) **EP 2 290 353 A1**
(43) Veröffentlichungstag der Anmeldung: **02.03.2011**
(21) Anmeldenummer: 09011195.6
(22) Anmeldetag: 01.09.2009
(51) Int. Cl.: G01N 21/85, G01N 29/02, G01N 33/28, H01F 27/40

(54) **Messanordnung zur Überwachung vom Transformatoröl**

(71) Anmelder: ABB Technology AG, 8050 Zürich (CH)
(72) Erfinder: Werle, Peter, 29664 Walsrode (DE); Szczechowski, Janusz, 04229 Leipzig (DE)
(74) Vertreter: Miller, Toivo

(57) **Zusammenfassung**

Die Erfindung betrifft eine Messanordnung (52, 60, 100, 120) zur Überwachung des Zustandes von Transformatoröl (12) eines Leistungstransformators (14), mit wenigstens einem Sensor (22, 70, 110) zur Messung einer auf diesen (22, 70, 110) auftreffenden Strahlung (26, 82, 106), mit einer direkt oder indirekt auf den Sensor (22, 70, 110) gerichteten Emissionsquelle (20, 68, 102) für die Abgabe einer definierten Strahlung (26, 82, 106) und mit einer Analysevorrichtung (28, 84) zur Ermittlung des Zustandes des Transformatoröls (12) anhand einer Wertedifferenz der auf den Sensor (22, 70, 110) auftreffenden Strahlung zur emittierten Strahlung. Zwischen der Emissionsquelle (20, 68, 102) und dem Sensor (22, 70, 110) ist zumindest abschnittsweise ein Raum (48, 74, 108) für eine Durchflutung mit Transformatoröl (12) vorgesehen. Die Strahlungsrichtung der Emissionsquelle (20, 68, 102) ist in den Innenraum eines den elektrischen Leistungstransformator (14) umgebenden ölgefüllten Transformatorkessels (16) gerichtet.

## Beschreibung

Die Erfindung betrifft eine Messanordnung zur Überwachung des Zustandes von Transformatoröl eines Leistungstransformators, mit wenigstens einem Sensor zur Messung einer auf diesen auftreffenden Strahlung, mit einer direkt oder indirekt auf den Sensor gerichteten Emissionsquelle für die Abgabe einer definierten Strahlung, mit einer Analysevorrichtung zur Ermittlung des Zustandes des Transformatoröls anhand einer Wertedifferenz der auf den Sensor auftreffenden Strahlung zur emittierten Strahlung, wobei zwischen Emissionsquelle und Sensor zumindest abschnittsweise ein Raum für eine Durchflutung mit Transformatoröl vorgesehen ist.

Es ist allgemein bekannt, dass elektrische Transformatoren für Energieverteilungsnetze, beispielsweise mit einer Nennleistung von 10 MVA bis mehrere 100 MVA und beispielsweise mit einer Nennspannung von 10 kV bis >400 kV, je nach Ausführungsform in einem mit Öl gefüllten zugehörigen Transformatorkessel angeordnet sind. Insbesondere Transformatoren im oberen Bereich des angegebenen Nennleis-tungsbandes sind als Öltransformatoren ausgeführt, wobei Transformatoren unterhalb des angegebenen Nennleistungsbandes häufig auch als Trockentransformatoren ausgeführt sind. Das Öl dient unter anderem als elektrisches Isolationsmedium aber auch als Kühlmedium zum Abführen von während des Transformatorbetriebes entstehender Verlustwärme.

Es ist auch bekannt, dass bei Öltransformatoren zur frühzeitigen Erkennung von Fehlern innerhalb des Transformators, beispielsweise einer fehlerhaften Isolation, regelmäßig Ölproben abgezapft werden, welche dann einer Analyse - beispielsweise in einem Chromatographen - zugeführt werden. Das Transformatoröl enthält nämlich eine Vielzahl von Indikationsfaktoren, welche Aufschluss über den Zustand des Transformators geben. Bei einer fehlerhaften Isolation zwischen zwei benachbarten Windungslagen einer Wicklung kann es beispielsweise zu Lichtbogenerscheinungen - auch mit Gasbildung - kommen, welche ihrerseits zu Einträgen in das Transformatoröl führen, was dem Fachmann jedoch bekannt ist.

Nachteilig hierbei ist, dass das Abzapfen der Ölproben einerseits mit einem hohen manuellen Aufwand verbunden ist und andererseits eine Untersuchung des Öls lediglich in bestimmten, vom zeitlichen Aufwand her vertretbaren Zeitintervallen erfolgt, beispielsweise alle 2 - 4 Monate, und eine kontinuierliche Ölanalyse nicht möglich ist.

Ausgehend von diesem Stand der Technik ist es Aufgabe der Erfindung, eine Vereinfachte Analysemöglichkeit für Ölproben bereitzustellen, welche die oben beschriebenen Nachteile vermeidet.

Diese Aufgabe wird gelöst durch eine Messanordnung der eingangs genannten Art. Diese ist **dadurch gekennzeichnet, dass** die Strahlungsrichtung der Emissionsquelle in den Innenraum eines den elektrischen Leistungstransformator umgebenden ölgefüllten Transformatorkessels gerichtet ist.

Unter Leistungstransformator sind in diesem Zusammenhang auch weitere elektrische Komponenten eines elektrischen Energieversorgungsnetzes zu verstehen, welche betriebsgemäß ebenfalls in einem ölgefüllten Kessel angeordnet sind, beispielsweise auch eine Drossel.

Der Grundgedanke der Erfindung liegt darin, einen Transport einer Ölprobe zu einem von dem Öltransformator entfernten Analyselabor zu vermeiden und eine Möglichkeit bereitzustellen, eine Ölanalyse direkt vor Ort durchzuführen. Eine geeignete Möglichkeit zur Vermeidung eines Öltransportes besteht darin, eine Analyse direkt im Transformatoröl, welches im Transformatorkessel befindlich ist, durchzuführen.

Besonders geeignet ist hierfür ein Messprinzip, bei welchem von einer Strahlungsquelle eine auf einen Sensor gerichtete Strahlung emittiert wird, welche sich dann im Öl ausbreitet und je nach Zustand des Öls - beispielsweise Trübung, Eintrag von Gasen oder Schwebstoffen - modifiziert und/oder abgeschwächt wird, wobei die auf den Sensor auftreffende Reststrahlung erfasst und quantifiziert wird. Ein Vergleich der erfassten Werte der auftreffenden Strahlung mit den Werten der von der Emissionsquelle abgegebenen Strahlung lässt - unter anderem auch unter Berücksichtigung des Abstandes zwischen Emissionsquelle und Sensor sowie auch optional unter Berücksichtigung weiterer Parameter wie beispielsweise der Öltemperatur - Aufschluss auf den Zustand des Transformatoröls und damit auch Aufschluss auf eventuelle schleichende Fehler innerhalb des Transformators zu.

Bei einer derartigen Messmethode werden analyseverursachte schädliche Eintragungen von Fremdstoffen in das Transformatoröl vermieden, wie es beispielsweise bei einer Gaschromatographie der Fall ist, wo die Ölprobe mit einem Trägergas wie Argon versetzt wird. Dies würde beispielsweise die Isolationsfähigkeit des Transformatoröls in negativer Weise reduzieren.

Zur Umsetzung einer derartigen Messmethode ist es vorteilhaft, die Emissionsquelle und/oder den Sensor möglichst in direktem Kontakt mit dem Transformatoröl zu bringen, so dass die Strahlung zwischen diesen Komponenten möglichst überwiegend im Transformatoröl verläuft und das Risiko von Messfehlern so reduziert wird. Ein direkter Kontakt einer Emissionsquelle und/oder eines Sensors, welches beide vorzugsweise elektrisch betriebene Komponenten sind, mit Transformatoröl ist jedoch zu vermeiden, auch um deren Beschädigung zu verhindern.

Von daher wird erfindungsgemäß vorgeschlagen, die Emissionsquelle und/oder den Sensor mit einer jeweiligen Kapselung zu umgeben, durch welche ein direkter Kontakt des Transformatoröls mit diesen Komponenten einerseits vermieden ist und wobei die Kapselung zumindest längs der Ausbreitungsrichtung der gerichteten Strahlung für diese durchlässig ist, beispielsweise Glas bei einer optischen Strahlung. Andererseits ist bei möglichst unmittelbarer Anordnung der Emissionsquelle und/oder des Sensors an der strahlungsdurchlässigen Wandung der Kapselung ein weitestgehend durch das Transformatoröl führender Strahlungsverlauf gewährleistet.

Somit ist in vorteilhafter Weise eine Analysemöglichkeit für Transformatoröl geschaffen, welche das Zapfen einer Ölprobe oder einen Transport des Öls vermeidet und zudem eine kontinuierliche Analyse des Zustandes des Transformatoröls ermöglicht.

In einer bevorzugten Ausgestaltung der erfindungsgemäßen Messanordnung sind der wenigstens der Sensor und/oder die Emissionsquelle derart gekapselt, dass diese komplett in Transformatoröl eintauchbar sind, ohne dass daraus deren Beschädigung resultiert. Eine derartige Vollkapselung ermöglicht eine maximale Flexibilität bei der Anordnung der Komponenten der erfindungsgemäßen Messanordnung.

Die Anordnung von Emissionsquelle und/oder Sensor erfolgt vorzugsweise innerhalb eines den Leistungstransformator umgebenden und mit Transformatoröl gefüllten Transformatorkessels. Der Zustand des dort befindlichen Transformatoröls gibt den höchsten Aufschluss über den Zustand des Transformators, weil hier der Abstand zum Transformator bzw. dessen Wicklungen am geringsten ist, im Gegensatz beispielsweise zu einem Ölausdehnungsgefäß. Fehler im Transformator und damit auch Einträge beispielsweise von Gas in das Transformatoröl treten nämlich überwiegend in den Wicklungen auf.

In einer besonders bevorzugten Ausführungsform der Messanordnung ist die Emissionsquelle ein Laser und die Strahlung ein Laserstrahl. Dies hat nämlich den Vorteil, dass die Strahlung sehr scharf fokussierbar und monofrequent ist und bedarfsweise auch in einer höheren Strahlungsintensität erzeugbar ist. Auf diese Weise ist ein systembedingtes Streuen der Strahlung von der Emissionsquelle zum Sensor weitestgehend vermieden. Jegliche Abschwächung oder Modifikation der Strahlung ist daher auf den jeweiligen Zustand des Transformatoröls zurückzuführen. Die erzielbare Analysegenauigkeit wird daher in vorteilhafter Weise gesteigert.

In einer weiteren Variante der erfindungsgemäßen Messanordnung ist mit dieser eine Laser-Flüssigkeitschromatographie durchführbar.

Für eine Chromatographie als solche sind eine stationäre Phase eines Messmediums, eine Herstellung eines Flusses einer mobilen Phase eines Messmediums, eine Injektion einer zu trennenden Probe, eine eigentliche Trennung und eine Detektion nötig, was dem Fachmann jedoch bekannt ist. Die Detektion kann insbesondere auch aufgrund von Lichtstreuung oder Lichtabsorption erfolgen.

Die Flüssigchromatographie ist als Spezialgebiet der Chromatographie eine physikalische Trennmethode. Als mobile und stationäre Phase kann hier jeweils eine Flüssigkeit, insbesondere auch Transformatoröl, dienen.

Unter Berücksichtigung der Tatsache, dass das Transformatoröl in einem Öltransformator im normalen Betrieb ohnehin aufgrund thermischer Strömungen in einem Fluss ist, erweist sich eine Flüssigkeitschromatographie, insbesondere eine laserbasierte, als besonders vorteilhaft für eine Analyse des Transformatoröls.

In einer weiteren Ausgestaltung der erfindungsgemäßen Messanordnung wird als Emissionsquelle eine Ultraschallquelle verwendet. Es hat sich nämlich gezeigt, dass auch eine Streuung bzw. eine Absorption von Ultraschall, welcher sich durch eine Flüssigkeit wie Transformatoröl ausbreitet, geeignet ist, Rückschlüsse auf den Zustand des Transformatoröls zu ziehen.

Eine bevorzugte Ausgestaltungsform der erfindungsgemäßen Messanordnung ist **dadurch gekennzeichnet, dass** die Strahlung der Emissionsquelle auf wenigstens einen Reflektor gerichtet ist, welcher die Strahlung in Richtung des Sensors ablenkt.

Dies ermöglicht eine flexible Strahlführung durch die zu untersuchenden Bereiche im Transformatorkessel. Je nach Ausführungsform des Öltransformators können auch mehrere voneinander entfernte Bereiche im Transformatorkessel von hohem Interesses für eine Analyse sein, beispielsweise im oberen horizontalen Bereich des Transformatorkessels, weil sich dort aufgrund der thermischen Bewegung des Öls das relativ wärmste Öl ansammelt. Ebenso von Interesse kann beispielsweise auch die Untersuchung von abgekühltem Transformatoröl sein, welches in einer vertikalen Abwärtsbewegung innerhalb des Transformatorkessels ist.

Mit einem Reflektor ist es somit in vorteilhafter Weise möglich, einen Laserstrahl zwischen Emissionsquelle und Sensor beispielsweise sowohl horizontal als auch vertikal zu führen und so auch voneinander entfernte relevante Untersuchungsbereiche zu analysieren.

In einer besonders bevorzugten Ausführungsform der Messanordnung mit Reflektor sind die Emissionsquelle und der Sensor dem Reflektor genau gegenüber liegend angeordnet. Dies ermöglicht eine kompaktere Bauform, bei der die aktiven Komponenten Emissionsquelle und Sensor beispielsweise innerhalb ein und derselben Kapselung angeordnet sind, wodurch der konstruktive Aufwand reduziert ist. Ein Reflektor ist normalerweise nämlich nicht zu kapseln.

In einer weiteren bevorzugten Variante der erfindungsgemäßen Messanordnung sind der Raum für eine Durchflutung mit Transformatoröl, der Sensor, die Emissionsquelle und - sofern vorhanden - der Reflektor längs einer gemeinsamen Linie angeordnet, so dass dieser Teil der Anordnung in einer lanzenähnliche Struktur ausgeformt ist.

Dies bietet den Vorteil einer besonders einfachen Einbringung dieser lanzenähnlichen Struktur in einen Transformatorkessel. Üblicherweise sind nämlich ohnehin verschließbare rohrähnliche Öffnungen durch die Wandung eines Transformatorkessels vorgesehen, nämlich für das Abzapfen von Ölproben. Eine lanzenähnliche Struktur der beschriebenen Komponenten ist daher besonders einfaches durch eine derartige Öffnung zu schieben und so in den Transformatorkessel einzubringen.

In einer weiteren Ausgestaltung der Messanordnung ist der Raum für eine Durchflutung mit Transformatoröl verschließ- und öffenbar. Der zu durchflutende Raum, durch welchen sich die emittierte Strahlung zum Sensor ausbreitet, muss während des Betriebes der Messanordnung systembedingt offen, also von Öl durchflutbar sein.

Auf der anderen Seite besteht beim Einführen einer lanzenähnlichen Struktur der betreffenden Komponenten der Messanordnung in den Transformatorkessel das Risiko, dass ungewollt Öl aus dem Transformatorkessel durch die Öffnung in der Kesselwandung nach außen gelangt, nämlich dann, wenn der zu durchflutende Raum gerade die Kesselwandung passiert. Ein bedarfsweises Verschließen dieses Raumes verhindert ein derartiges Auslaufen in vorteilhafter Weise. Es ist aber auch denkbar, durch längs in Einführrichtung verlaufende Dichtungslippen diesen Raum indirekt durch die Dichtungslippen zu verschließen. Hierzu ist allerdings die axiale Erstreckung der Dichtungslippen länger auszuführen als die axiale Erstreckung des zu durchflutenden Raumes.

Dichtungslippen als solche oder vergleichbare Mittel sind in der erfindungsgemäßen Messanordnung in jedem Fall vorteilhaft, um so eine öldichten Verbindung der komplett eingeführten lanzenähnlichen Struktur mit der Wandung zu ermöglichen.

Die erfindungsgemäße Aufgabe wird auch gelöst durch einen Transformator mit Messanordnung nach einem der Ansprüche 9 bis 11, wobei die Messanordnung zumindest teilweise - wenigstens aber mit dem Raum für eine Durchflutung mit Transformatoröl und wenigstens mit einer der Komponenten Sensor, Emissionsquelle oder Reflektor - durch eine verschließbare Öffnung in der Wandung des den Leistungstransformator umgebenden Transformatorkessels in dessen mit Transformatoröl gefülltes Inneres einführbar ist.

Die erfindungsgemäßen Vorteile entsprechen den zuvor genannten Vorteilen. Bezüglich der Einführung von Komponenten in den Transformatorkessel ist systembedingt wenigstens der zu durchflutende Raum in dem Kesselinneren anzuordnen. An diesen Raum grenzen durch das Messprinzip bedingt an zwei gegenüberliegenden Seiten jeweils wenigstens eine der Komponenten Emissionsquelle, Sensor oder Reflektor an. Deshalb ist wenigstens eine dieser Komponenten komplett in Transformatoröl getaucht, nämlich die am weitesten in den Transformatorkessel hereinragende Komponente.

Die wenigstens eine gegenüberliegende Komponente ist nicht zwangsläufig komplett in Öl getaucht, vielmehr kann diese schon durch Teile der verschließbaren Öffnung geschützt sein, so dass in diesem Fall auch eine Teilkapselung der Komponente zum Schutz vor Transformatoröl ausreichend ist.

Vorzugsweise ist die in das Innere des Transformatorkessels eingeführte Messanordnung öldicht und lösbar mit der Wandung des Transformatorkessels verbindbar. Hierdurch ist beispielsweise auch der mobile Einsatz einer erfindungsgemäßen Messanordnung ermöglicht, wenn beispielsweise aufgrund einer bevorstehenden Wartung bereits im Voraus kontinuierlich eine Ölanalyse durchzuführen ist und eine Messanordnung nur zeitweise benötigt wird.

Weitere vorteilhafte Ausgestaltungsmöglichkeiten sind den weiteren abhängigen Ansprüchen zu entnehmen.

Anhand der in den Zeichnungen dargestellten Ausführungsbeispiele sollen die Erfindung, weitere Ausführungsformen und weitere Vorteile näher beschrieben werden.

Es zeigen:
- Fig. 1: einen Transformator mit exemplarischer erster Messanordnung,
- Fig. 2: einen Transformator mit exemplarischer zweiter Messanordnung,
- Fig. 3: eine exemplarische dritte Messanordnung in einer Wandung,
- Fig. 4: eine exemplarische vierte Messanordnung in einer Wandung,
- Fig. 5: eine exemplarische fünfte Messanordnung in einer Wandung und
- Fig. 6: eine Wandung mit Verschlusselementen.

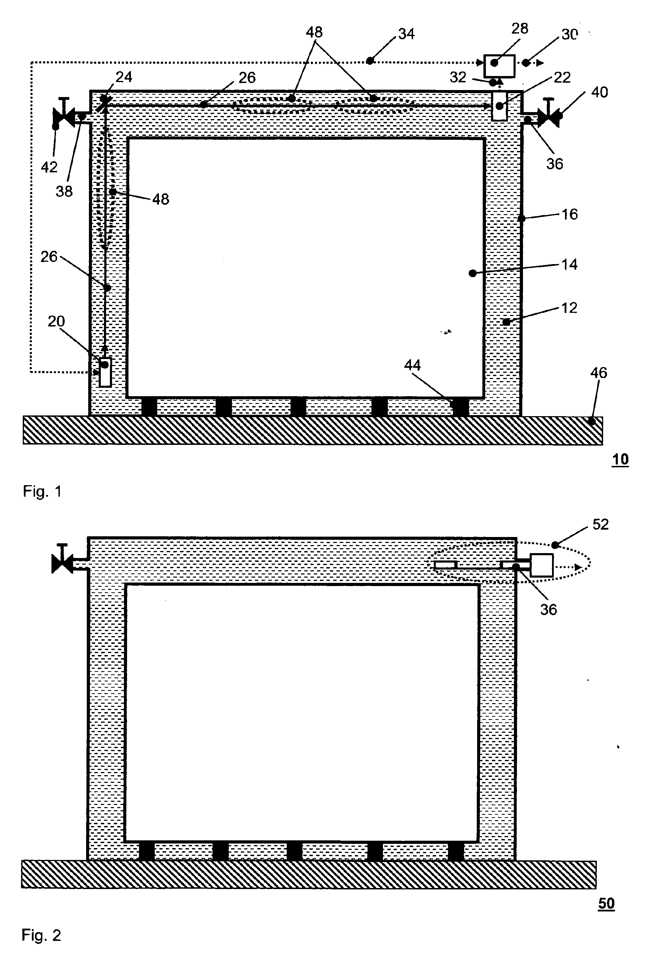

Fig. 1 zeigt einen Transformator mit einer exemplarischen Messanordnung 10. Ein elektrischer Transformator 14, also im Wesentlichen ein Transformatorkern mit Wicklungen, ist auf Distanzelementen 44 stehend annähernd mittig in einem Transformatorkessel 16 angeordnet, welcher komplett mit Transformatoröl 12 geflutet ist. Im oberen Bereich des Transformatorkessels 16 sind in dessen Wandung eine erste 36 und eine zweite 38 Öffnung vorgesehen, welche rohrähnlich nach außen geführt und mit einem ersten 40 bzw. zweiten 42 Öffnungsverschluss, beispielsweise einem Zapfhahn, versehen sind. Derartige Zapfhähne 40, 42 sind bei Öltransformatoren üblich und dienen dazu, ein Abzapfen von Ölproben zu Analysezwecken zu ermöglichen, wobei die Analyse üblicherweise in einem entfernten Analyselabor erfolgt.

Innerhalb des mit Transformatoröl 12 gefluteten Transformatorkessels 16 ist zwischen Kesselwandung und elektrischem Transformator 14 im unteren linken Bereich eine erste Emissionsquelle 20, beispielsweise ein Laser, angeordnet. Dieser ist vorzugsweise gekapselt, so dass eine Beschädigung durch das Transformatoröl, in welches er komplett getaucht ist, vermieden ist. Die Emissionsquelle 20 bzw. der Laser emittiert eine erste gebündelte Strahlung 26, in diesem Fall einen Laserstrahl 26, vertikal nach oben gegen den ersten Reflektor 24, beispielsweise einen Spiegel, welcher den Laserstrahl 26 horizontal nach rechts gegen den im rechten oberen Bereich des Transformatorkessels 16 angeordneten Sensor 22 lenkt.

Der Laserstrahl 26 quert hierbei einen öldurchfluteten Raum innerhalb des Transformatorkessels, welcher in mehreren Bereichen mit der Bezugsziffer 48 angedeutet ist. In diesem Fall ist der öldurchflutete Raum 48 kein separater, abgegrenzter Raum, vielmehr ist er Teil des gesamten vom Transformatoröl 12 durchfluteten Innenraums des Transformatorkessels 16. Während der Ausbreitung des Laserstrahls 26 von der Emissionsquelle 20 bis zum ersten Sensor 22 durch das thermisch geringfügig bewegte Transformatoröl 12 wird er beispielsweise abgeschwächt und auch teilweise gestreut.

Der auf den ersten Sensor 22 auftreffende Laserstrahl 26 wird von diesem erfasst und dessen Eigenschaften in quantifizierten Messdaten 32 der ersten Analysevorrichtung 28 zur Verfügung gestellt. Die erste Analysevorrichtung 28 steuert über die erste Steuerleitung 34 sowohl die Intensität der ersten Emissionsquelle 20 und bekommt in diesem Beispiel auch eine Rückmeldung über die Tatsächliche Intensität des Laserstrahls 26. Selbstverständlich ist auch eine gepulste oder nur zeitweilige Ansteuerung der Emissionsquelle möglich. Anhand der Differenz des emittierten Laserstrahls und des an dem ersten Sensor auftreffenden Laserstrahls wird mittels der ersten Analysevorrichtung 28 der Zustand des Transformatoröls 12 ermittelt, welcher letztendlich Aufschluss über den Zustand des in diesem befindlichen Transformators 14 gibt. Die Ergebnisse der Analyse werden als Ausgabedaten 30 weiteren, nicht gezeigten, Kontrollkomponenten zur Verfügung gestellt werden. Die Ausgabedaten 30 können sowohl mittels einer Datenleitung aber beispielsweise auch drahtlos über einen Sender bereitgestellt werden.

Vorzugsweise erfolgt die Analyse des Transformatoröls 12 nach dem bekannten Prinzip der Laser-Flüssigkeitschromatographie in sich über einen bestimmten Zeitraum erstreckenden Messintervallen, so dass insbesondere auch die Strömung des Transformatoröls berücksichtigt ist, welche sich aus thermischen Effekten während des Abwärme produzierenden Betriebes des Transformators 14 ergibt. Weitere Details des Transformators 14 bzw. des Transformatorkessels 16, wie beispielsweise ein Ölausdehnungsgefäß oder ein Lüfter, sind in der Fig. nicht gezeigt aber als vorhanden anzunehmen.

Fig. 2 zeigt einen baugleichen Transformator wie in der vorherigen Fig., es ist nunmehr aber anstelle der zuvor beschriebenen Komponenten der ersten Messanordnung eine lanzenähnliche zweite Messanordnung 52 über die erste Öffnung 36 in das Innere des Transformatorkessels eingeführt. Mögliche Varianten für die zweite Messanordnung 52 sind den nachfolgenden Fig. in einer Detailansicht zu entnehmen.

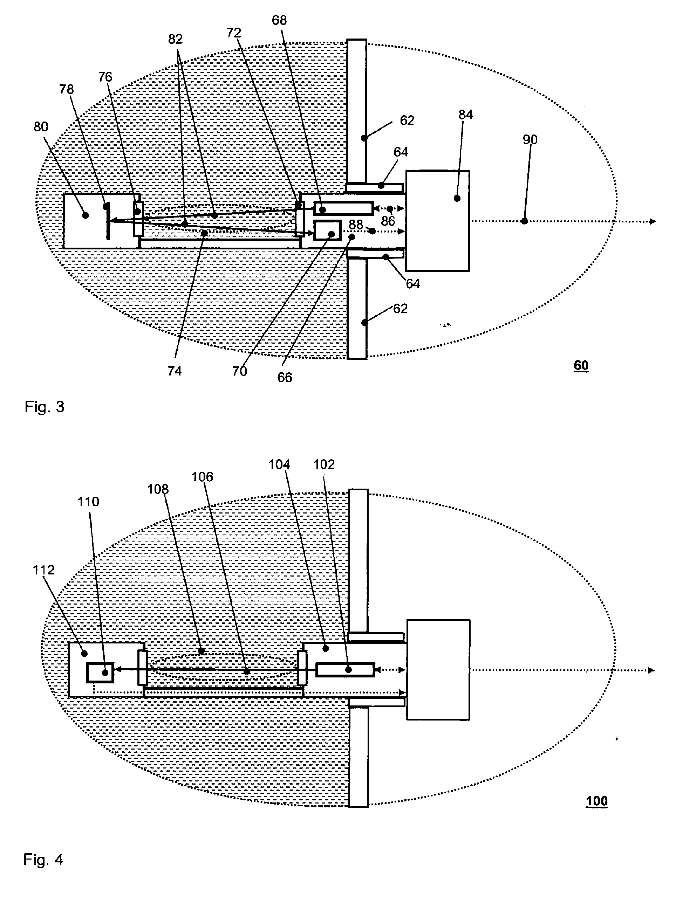

Fig. 3 zeigt eine exemplarische dritte Messanordnung 60 in der Öffnung einer Wandung 62 eines Transformatorkessels. Eine zweite Emissionsquelle 68, ebenfalls ein Laser, und ein zweiter Sensor 70 sind in einem gemeinsamen gekapselten ersten Raum 66 angeordnet. Dieser bietet einen Schutz gegen das ihn umgebende Transformatoröl. An seiner in der Fig. links dargestellten Seite ist eine erste transparente Scheibe 72 vorgesehen, welche einerseits einen Schutz der Emissionsquelle 68 und des Sensors 70 gegen das Transformatoröl darstellt, aber andererseits durchlässig für die abgegebene und die auftreffende zweite Strahlung 82 ist, in diesem Fall ein Laserstrahl 82.

Die von der zweiten Strahlungsquelle 68 abgegebene Laserstrahlung 82 breitet sich annähernd längs einer senkrecht zur Wandung 62 verlaufenden Achse aus und durchquert nach Durchtritt durch die erste transparente Scheibe 72 einen Raum 82 für die Durchflutung mit Transformatoröl. Dort wird die Laserstrahlung entsprechend des Zustandes des Transformatoröls, mit welchen der Raum 82 geflutet ist, abgeschwächt, gestreut oder anderweitig verändert. Nun trifft die Strahlung durch eine zweite transparente Scheibe 76 in einen ebenfalls längs der senkrecht zur Wandung 62 verlaufenden Achse angeordneten zweiten gekapselten Raum 82, in welchem ein zweiter Reflektor 78, in diesem Fall ein Spiegel, angeordnet ist.

Dieser Spiegel 78 reflektiert den eintreffenden Laserstrahl 82 nun in einem Winkel von annähernd 180° zurück, so dass der ebenfalls annähernd längs der Achse verlaufende reflektierte Laserstrahl auf den zweiten Sensor 70, welcher direkt benachbart zur zweiten Emissionsquelle 68 angeordnet ist, trifft. Eine Kapselung des zweiten Reflektors 78 ist nicht zwangsläufig notwendig, vielmehr ist es auch möglich den Reflektor mit einer entsprechenden Haltevorrichtung direkt in dem Transformatoröl anzuordnen.

Der erste gekapselte Hohlraum 66, der mit Öl durchflutbare Raum 82 und der zweite gekapselte Hohlraum 80 sind längs einer Linie angeordnet, so dass diese Komponenten bei einem vorzugsweise runden Querschnitt eine lanzenähnliche Struktur bilden, welche besonders einfach durch eine Öffnung in der Wandung 62 eines Transformatorkessels in dessen Inneres einführbar sind. In der Fig. ist der Rand der als kreisrund anzunehmenden Öffnung mit einem ebenfalls als kreisrund anzunehmenden Dichtelement 64 versehen, beispielsweise einer Dichtlippe, durch welche ein Auslaufen von Öl durch die Öffnung verhindert ist.

Eine zweite Auswertevorrichtung 84 ist an dem in der Fig. rechts dargestellten Bereich der lanzenähnlichen Struktur angeordnet. Analog zu der zuvor gezeigten Fig. ist diese mit der zweiten Emissionsvorrichtung 68 über eine bidirektionale zweite Steuerleitung 86 verbunden und bekommt von dem zweiten Sensor 70 Messdaten 88 zur Verfügung gestellt. Nach einer Analyse des Transformatoröls werden die Ausgabedaten 90 dann weiteren Kontrollkomponenten zur Verfügung gestellt.

Fig. 4 zeigt eine exemplarische vierte Messanordnung 100 in der Öffnung einer Wandung desselben Transformatorkessels wie bei der vorherigen Fig.. Diese weist ebenfalls eine lanzenähnliche Struktur von längs einer Linie angeordneten Elementen, nämlich drittem gekapselten Hohlraum 104, durchflutbarem Raum 108 und viertem gekapselten Hohlraum 112 auf, so dass auch vierte Messanordnung analog zu Fig. 3 durch die Öffnung einführbar ist. Im Unterschied zu der zuvor gezeigten Fig. wird aber auf einen Reflektor verzichtet, vielmehr ist in dem dritten gekapselten Hohlraum 104 lediglich eine dritte Emissionsquelle 102 angeordnet, welche eine dritte Strahlung 106 auf einen im vierten gekapselten Hohlraum angeordneten vierten Sensor 112 abgibt.

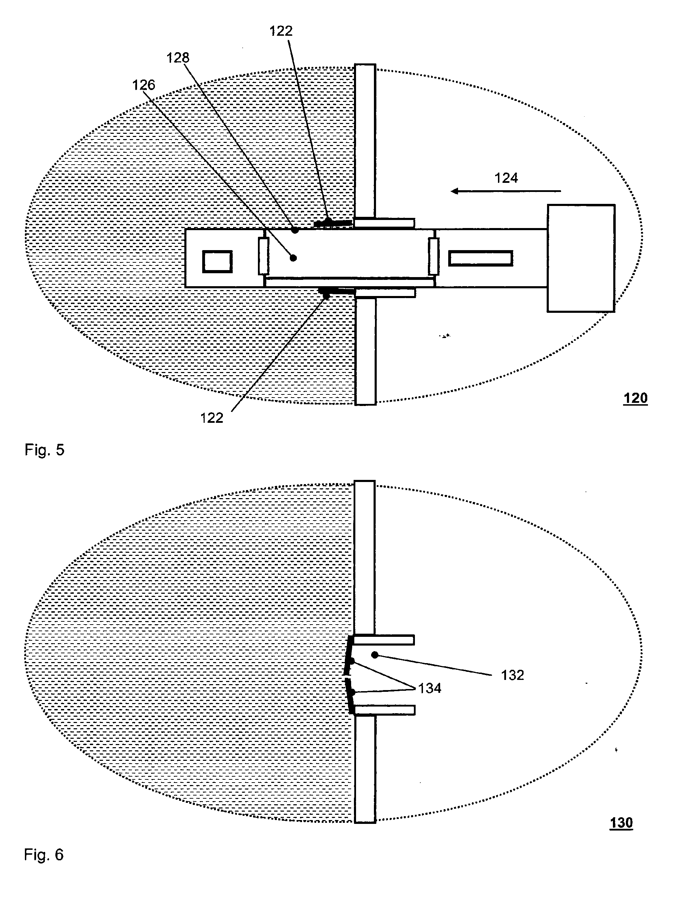

Fig. 5 zeigt eine exemplarische fünfte Messanordnung 120, welche der vierten Messanordnung 100 aus Fig. 4 entspricht, in der Öffnung einer Wandung desselben Transformatorkessels wie bei den vorherigen Fig. in einem noch nicht komplett eingeführten Zustand. Der Raum 126 für die Durchflutung mit Transformatoröl ist nunmehr aber mit einem Verschluss 128 verschlossen, beispielsweise mit einer beweglichen Zylinderschale. Somit ist verhindert, dass in der dargestellten Phase der Einführung der lanzenähnlichen Struktur in einen mit Öl gefüllten Transformatorkessel Öl durch den Hohlraum 126 ausläuft. Ferner gezeigt ist noch ein klappbares Verschlusselement 122, welches den vorzugsweise kreisrunden Querschnitt der lanzenähnlichen Struktur umschließt und bei komplett herausgezogener lanzenähnlicher Struktur die Öffnung der Wandung öldicht verschließt.

Fig. 6 zeigt die Wandung der vorherigen Fig. ohne Messvorrichtung aber mit einer durch Verschlusselemente 134 verschlossenen Öffnung 132. Durch die Verschlusselemente 134 ist ein Herauslaufen des Transformatoröls bei nicht vorhandener Messvorrichtung sichergestellt.

### Bezugszeichenliste

- 10: Transformator mit exemplarischer erster Messanordnung
- 12: Transformatoröl
- 14: Transformator
- 16: Transformatorkessel
- 20: erste Emissionsquelle
- 22: erster Sensor
- 24: erster Reflektor
- 26: erste Strahlung
- 28: erste Analysevorrichtung
- 30: Ausgabedaten der ersten Analysevorrichtung
- 32: Messdaten des ersten Sensors
- 34: erste Steuerleitung
- 36: erste Öffnung in der Wandung des Transformatorkessels
- 38: zweite Öffnung in der Wandung des Transformatorkessels
- 40: erster Öffnungsverschluss
- 42: zweiter Öffnungsverschluss
- 44: Distanzelement
- 46: Standfläche
- 48: Raum für die Durchflutung mit Transformatoröl
- 50: Transformator mit exemplarischer zweiter Messanordnung
- 52: exemplarische zweite Messanordnung
- 60: exemplarische dritte Messanordnung in Wandung
- 62: Wandung
- 64: Dichtelement
- 66: erster gekapselter Raum
- 68: zweite Emissionsquelle
- 70: zweiter Sensor
- 72: erste transparente Scheibe
- 74: Raum für die Durchflutung mit Transformatoröl
- 76: zweite transparente Scheibe
- 78: zweiter Reflektor
- 80: zweiter gekapselter Raum
- 82: zweite Strahlung
- 84: zweite Analysevorrichtung
- 86: zweite Steuerleitung
- 88: Messdaten des zweiten Sensors
- 90: Ausgabedaten der zweiten Analysevorrichtung
- 100: exemplarische vierte Messanordnung in Wandung
- 102: dritte Emissionsquelle
- 104: dritter gekapselter Hohlraum
- 106: dritte Strahlung
- 108: Raum für die Durchflutung mit Transformatoröl
- 110: dritter Sensor
- 112: vierter gekapselter Hohlraum
- 120: exemplarische fünfte Messanordnung in Wandung
- 122: Verschlusselement
- 124: Bewegungsrichtung
- 126: verschlossener Raum für die Durchflutung mit Transformatoröl
- 128: Verschluss für Raum für die Durchflutung mit Transformatoröl
- 130: Wandung mit Verschlusselementen
- 132: Öffnung in Wandung
- 134: Verschlusselement

## Patentansprüche

1. Messanordnung (52, 60, 100, 120) zur Überwachung des Zustandes von Transformatoröl (12) eines Leistungstransformators (14), mit wenigstens einem Sensor (22, 70, 110) zur Messung einer auf diesen (22, 70, 110) auftreffenden Strahlung (26, 82, 106), mit einer direkt oder indirekt auf den Sensor (22, 70, 110) gerichteten Emissionsquelle (20, 68, 102) für die Abgabe einer definierten Strahlung (26, 82, 106), mit einer Analysevorrichtung (28, 84) zur Ermittlung des Zustandes des Transformatoröls (12) anhand einer Wertedifferenz der auf den Sensor (22, 70, 110) auftreffenden Strahlung zur emittierten Strahlung, wobei zwischen Emissionsquelle (20, 68, 102) und Sensor (22, 70, 110) zumindest abschnittsweise ein Raum (48, 74, 108) für eine Durchflutung mit Transformatoröl (12) vorgesehen ist, **dadurch gekennzeichnet, dass** die Strahlungsrichtung der Emissionsquelle (20, 68, 102) in den Innenraum eines den elektrischen Leistungstransformator (14) umgebenden ölgefüllten Transformatorkessels (16) gerichtet ist.

2. Messanordnung nach Anspruch 1, **dadurch gekennzeichnet, dass** wenigstens der Sensor (22, 70, 110) und/oder die Emissionsquelle (20, 68, 102) öldicht gekapselt (66, 80, 104, 112) sind, so dass diese in Transformatoröl (12) eintauchbar sind, ohne dass daraus deren Beschädigung resultiert.

3. Messanordnung nach einem der Ansprüche 1 oder 2, **dadurch gekennzeichnet, dass** wenigstens der Sensor (22, 70, 110) und/oder die Emissionsquelle (20, 68, 102) innerhalb des den Leistungstransformator (14) umgebenden und mit Transformatoröl (12) gefüllten Transformatorkessels (16) angeordnet sind.

4. Messanordnung nach einem der vorangegangenen Ansprüche, **dadurch gekennzeichnet, dass** die Emissionsquelle (20, 68, 102) ein Laser und die Strahlung (26, 82, 106) ein Laserstrahl ist.

5. Messanordnung nach Anspruch 4, **dadurch gekennzeichnet, dass** mit dieser eine Laser-Flüssigkeitschromatographie durchführbar ist.

6. Messanordnung nach einem der Ansprüche 1 bis 3, **dadurch gekennzeichnet, dass** die Emissionsquelle (20, 68, 102) eine Ultraschallquelle ist.

7. Messanordnung nach einem der vorangegangenen Ansprüche, **dadurch gekennzeichnet, dass** die Strahlung (26, 82, 106) der Emissionsquelle (20, 68, 102) auf wenigstens einen Reflektor (24, 78) gerichtet ist, welcher die Strahlung (26, 82, 106) in Richtung des Sensors (22, 70, 110) ablenkt.

8. Messanordnung nach Anspruch 7, **dadurch gekennzeichnet, dass** die Emissionsquelle (68) und der Sensor (70) dem Reflektor (78) genau gegenüber liegend angeordnet sind.

9. Messanordnung nach einem der vorangegangenen Ansprüche, **dadurch gekennzeichnet, dass** der Raum (48, 74, 108) für eine Durchflutung mit Transformatoröl (12), der Sensor (22, 70, 110), die Emissionsquelle (20, 68, 102) und - sofern vorhanden - der Reflektor (24, 78) längs einer gemeinsamen Linie angeordnet sind, so dass dieser Teil der Anordnung in einer lanzenähnliche Struktur ausgeformt ist.

10. Messanordnung nach Anspruch 9, **dadurch gekennzeichnet, dass** der Raum (48, 74, 108) für eine Durchflutung mit Transformatoröl (12) verschließ- und öffenbar (128) ist.

11. Messanordnung nach Anspruch 9 oder 10, **dadurch gekennzeichnet, dass** die lanzenähnliche Struktur zumindest teilweise durch eine Öffnung (36, 38, 132) einer Wandung einführbar ist und dass Mittel (64, 122, 134) zur öldichten Verbindung der eingeführten lanzenähnlichen Struktur mit der Wandung vorgesehen sind.

12. Transformator mit Messanordnung nach einem der Ansprüche 9 bis 11, **dadurch gekennzeichnet, dass** die Messanordnung zumindest teilweise - wenigstens aber mit dem Raum (48, 74, 108) für eine Durchflutung mit Transformatoröl (12) und wenigstens mit einer der Komponenten Sensor (22, 70, 110), Emissionsquelle (20, 68, 102) oder Reflektor (24, 78) - durch eine verschließbare Öffnung (36, 38, 132) in der Wandung des den Leistungstransformator (14) umgebenden Transformatorkessels (16) in dessen mit Transformatoröl (12) gefülltes Inneres einführbar ist.

13. Transformator mit Messanordnung nach Anspruch 12, **dadurch gekennzeichnet, dass** die in das Innere des Transformatorkessels (16) eingeführte Messanordnung (52, 60, 100, 120) öldicht und lösbar mit der Wandung des Transformatorkessels (16) verbindbar ist.
